# EUROPEAN PATENT SPECIFICATION

(11) **EP 3 366 252 B1**
(45) Date of publication and mention of the grant of the patent: **08.07.2020**
(21) Application number: 18157796.6
(22) Date of filing: 21.02.2018
(51) Int. Cl.: A61B 18/18, A61B 8/08, A61B 18/00, A61B 18/14, A61B 8/12, A61B 8/00

(54) **ULTRASOUND DOPPLER AND ELASTOGRAPHY FOR ABLATION PREDICTION AND MONITORING**
ULTRASCHALL-DOPPLER-VERFAHREN UND ELASTOGRAPHIE ZUR ABLATIONSVORHERSAGE UND -ÜBERWACHUNG
PROCEDÉE DOPPLER À ULTRASONS ET ÉLASTOGRAPHIE POUR LA PRÉDICTION ET LA SURVEILLANCE D'ABLATION

(30) Priority: 22.02.2017 US 201762462129 P; 15.02.2018 US 201815897803
(43) Date of publication of application: 29.08.2018
(73) Proprietor: Covidien LP, Mansfield, MA 02048 (US)
(72) Inventor: BRANNAN, Joseph D., Lyons, CO Colorado 80540 (US)
(74) Representative: Morgan, Marc

(56) References cited:
- WO-A1-95/29737
- WO-A1-2016/001783
- US-A1- 2015 305 821
- US-A1- 2016 206 373
- BRUNO D. FORNAGE: "Role of color Doppler imaging in differentiating between pseudocystic malignant tumors and fluid collections", JOURNAL OF ULTRASOUND IN MEDICINE, vol. 14, no. 2, 1 February 1995 (1995-02-01), pages 125-128, XP055473695,

## Description

### BACKGROUND

### Technical Field

The present disclosure relates to the use of ultrasound for ablation prediction and monitoring, and more specifically, to using the Doppler effect and elastography as detected by ultrasound to predict and monitor ablation zone shape, size, and progress.

### Description of Related Art

Ultrasound scans of a patient's body are commonly used to confirm placement of surgical tools at treatment locations inside the patient's body. However, once treatment commences, there exists no way to predict or confirm the area, scope, and/or progress of the treatment other than by using software models. The present disclosure provides improvements to the use of ultrasound scans during treatment procedures to predict and confirm the size, shape, and location of treatment zones and the progress of treatment procedures.

WO 9529737 discloses a method and apparatus for noninvasive and real-time monitoring and feedback control of the extent and geometry of tissue damage induced by various thermal modalities (laser, electromagnetic wave, ultrasound and thermistor) in different thermal therapies (hyperthermia, thermal coagulation and ablation). Unlike ultrasound configurations which use non-Doppler ultrasound techniques, this citation discloses a single-beam configuration that employs a multiple-range-gate pulsed Doppler technique. The configuration may be operated in A-mode, M-mode, or multi-dimensional image mode to monitor tissue thermal response in the tissue being treated at multiple tissue depths along the sound beam. By measuring changes in phase (i.e., motion) and amplitude (i.e., echogenicity) of the echoes returned from the tissue under treatment, the Doppler system can determine temporal and spatial profiles of tissue temperature and the extent and geometry of tissue thermal damage. The system can also differentiate tissue responses corresponding to coagulation of tissue (in hyperthermia or coagulation treatment) versus ablation of tissue. Further, the Doppler detection provides feedback signals using fuzzy logic technology to automatically and in real-time regulate thermal output of various thermal modalities so that optimal thermal treatment can be obtained. The control of thermal output is achieved by adjusting treatment parameters such as pulse rate, exposure time and output power in the case of lasers. The Doppler detection results can also be shown on a suitable display device to allow manual feedback control of a thermal modality by a human operator.

### SUMMARY

The invention is defined in the appended claims.

As described herein, a system for ablation zone detection includes an ablation device configured to deliver microwave energy to tissue at a treatment location, an ultrasound device configured to deliver ultrasound energy to the tissue, receive, from a plurality of respective portions of the tissue, a plurality of ultrasound return signals that are based on the delivered ultrasound energy, and detect, in the plurality of return signals, a plurality of respective Doppler shifts that are based on the microwave energy delivered to the tissue, a computing device configured to determine an ablation zone characteristic based on the plurality of Doppler shifts, and generate an image representing at least a portion of the tissue and a representation of the ablation zone characteristic, and a display device configured to display the image by way of a graphical user interface. The computing device is further configured to display on the display device an indication of the size and shape of the ablation zone on an ultrasound image.

As described herein, the plurality of Doppler shifts have a plurality of directions, respectively, and wherein the determining the ablation zone characteristic includes identifying one or more regions of the tissue that correspond to one or more of the plurality of Doppler shifts, respectively.

As described herein, the system further includes determining a plurality of characteristics of the plurality of Doppler shifts, wherein the plurality of characteristics includes any one or a combination of an amount of Doppler shift, a rate of change of the amount of Doppler shift, a direction of Doppler shift, a rate of change of the direction of Doppler shift, or a location in the tissue based upon which Doppler shift is detected, and determining whether each of the plurality of characteristics exceeds one or more thresholds, wherein the determining of the ablation zone characteristic is further based on a result of the determining of whether each of the plurality of characteristics exceeds the one or more thresholds.

As described herein, the determining of the ablation zone characteristic includes defining an ablation zone, the portions of the tissue that correspond to the plurality of Doppler shifts having respective characteristics that exceed the one or more thresholds being included in the ablation zone, and the portions of the tissue that correspond to the plurality of Doppler shifts having respective characteristics that do not exceed the one or more thresholds being excluded from the ablation zone, wherein the ablation zone characteristic includes the combination of the shape, the size, and the location of the ablation zone.

As described herein, the system further includes determining, if the ablation continues as planned, a predicted ablation zone characteristic based on any one or a combination of the

plurality of Doppler shifts, the determined ablation zone characteristic, a setting of the ablation device, an elapsed duration for which the microwave energy is delivered to the tissue, an expected duration for which the microwave energy will be delivered to the tissue, a location in the tissue to which the microwave energy is delivered, or one or more characteristics of a signal by which the microwave energy is delivered to the tissue, and displaying, by way of the graphical user interface, a representation of the predicted ablation zone characteristic.

As described herein, the one or more characteristics of the signal by which the microwave energy is delivered include any one or a combination of an amount of power of the signal, an amplitude of the signal, a frequency of the signal, a frequency of pulsing of the signal, or a width of pulsing of the signal.

As described herein, the system further includes determining an estimated amount of time until completion of an ablation procedure, based on any one or a combination of the plurality of Doppler shifts, the determined ablation zone characteristic, an elapsed duration for which the microwave energy is delivered to the tissue, a location in the tissue to which the microwave energy is delivered, or one or more characteristics of a signal by which the microwave energy is delivered, and displaying, via the graphical user interface, an indicator of the estimated amount of time until completion of the ablation procedure.

As described herein, the system further includes determining whether an ablation procedure is completed based on any one or a combination of the plurality of Doppler shifts, the determined ablation zone characteristic, an elapsed duration for which the microwave energy is delivered to the tissue, a location in the tissue to which the microwave energy is delivered, or one or more characteristics of a signal by which the

microwave energy is delivered, and displaying, via the graphical user interface, an indicator of whether the ablation procedure is completed.

As described herein, the system further includes automatically disabling the delivering of the microwave energy to the tissue in response to a determination that the ablation procedure is completed.

As described herein, the system further includes determining a percentage of completion of an ablation procedure based on any one or a combination of the plurality of Doppler shifts, the determined ablation zone characteristic, an elapsed duration for which the microwave energy is delivered to the tissue, a location in the tissue to which the microwave energy is delivered, or one or more characteristics of a signal by which the microwave energy is delivered, and displaying, via the graphical user interface, an indicator of the percentage of completion of the ablation procedure.

As described herein, the system further includes automatically adjusting, by way of one or more respective settings of the ablation device, one or more characteristics of a microwave signal by which the microwave energy is delivered to the tissue based on the percentage of completion of the ablation procedure.

As described herein, the ablation zone characteristic includes one or more of an ablation zone shape, an ablation zone size, or an ablation zone location.

As described herein, the representation of the ablation zone characteristic includes a uniformly-colored or uniformly-shaded shape superimposed, by way of the graphical user interface, upon a portion of the image that corresponds to an ablation zone.

As described herein, the representation of the ablation zone characteristic includes a shape having a plurality of levels of shading or coloring corresponding to a plurality of respective levels of a characteristic of the plurality of Doppler shifts, wherein the shape is superimposed, by way of the graphical user interface, upon a corresponding portion of the image.

As described herein, the plurality of Doppler shifts include a first Doppler shift having a first direction and a second Doppler shift having a second direction, and the representation of the ablation zone characteristic includes speckles of a first color and speckles of a second color, wherein the speckles of the first color correspond to locations in the tissue based upon which the first Doppler shift is detected, and the speckles of the second color correspond to locations in the tissue based upon which the second Doppler shift is detected.
As described herein, the microwave energy and the ultrasound energy are simultaneously delivered to the tissue.

### BRIEF DESCRIPTION OF THE DRAWINGS

Various aspects and features of the present disclosure are described hereinbelow with references to the drawings, wherein:
FIG. 1 is a schematic diagram of a system for using ultrasound Doppler and elastography to predict and monitor ablation, according to an embodiment of the present disclosure;
FIG. 2 is a diagram of an exemplary computing device forming part of the system of FIG. 1, according to an embodiment of the present disclosure;
FIG. 3 shows a flowchart of an exemplary method for using Doppler effect to predict a size and shape of an ablation zone;
FIG. 4 shows another flowchart of an exemplary method for using Doppler effect to predict a size and shape of an ablation zone;
FIG. 5 shows a flowchart of an exemplary method for using elastography to monitor a size and shape of an ablation zone;
FIG. 6 shows another flowchart of an exemplary method for using elastography to monitor a size and shape of an ablation zone;
FIG. 7 shows an exemplary graphical user interface which may be used to display and monitor a size and shape of an ablation zone, according to an embodiment of the present disclosure;
FIG. 8 shows another exemplary graphical user interface which may be used to display and monitor a size and shape of an ablation zone, according to an embodiment of the present disclosure; and
FIG. 9 shows yet another exemplary graphical user interface which may be used to display and monitor a size and shape of an ablation zone, according to an embodiment of the present disclosure.

### DETAILED DESCRIPTION

The present disclosure is directed to devices and systems for predicting, detecting, and monitoring ablation zones. More particularly, the disclosure relates to using the Doppler effect and elastography, as detected by ultrasound, to predict, detect, and monitor ablation zone shape, size, and progress. The prediction, detection, and monitoring may be performed using one or more software applications executed on a computer.

The Doppler effect is the change in perceived frequency or wavelength of a signal moving relative to its source. The frequency or wavelength of the signal does not actually change, but it is perceived to change due to movement of its source. For example, the frequency of the signal will be perceived as higher while the source is moving towards a detector, and will be perceived as lower while the source is moving away from the detector. When an ultrasound sensor is used to detect tissue vibrations, the vibrating tissue may be the source of the signals, and areas of higher frequency (indicating movement toward the ultrasound sensor) may be depicted as blue speckles, while areas of lower frequency (indicating movement away from the ultrasound sensor) may be depicted as red speckles. In order to predict the size and shape of the ablation zone based on a detected Doppler effect, the application may, for example, receive ultrasound data from an ultrasound imaging system operating in a mode wherein the ultrasound imaging system is configured to detect the Doppler effect.

The application may then process the received ultrasound data to identify an area where a Doppler shift is occurring, that is, and area where tissue is vibrating and thus causing a red-and-blue speckled cloud detectable in the ultrasound data, the red-and-blue speckled cloud being indicative of the size and shape of the ablation zone. The application may further cause a display device to display an indication of the size and shape of the ablation zone on ultrasound images, based on the size and shape of the red-and-blue speckled cloud. It should be noted that a Doppler shift may be present and detected in multiple areas of a patient's body, including areas where an ablation is not being performed. However, a Doppler shift profile will be unique when caused by tissue heating from an ablation device, and thus easily detectable, in tissue that is being ablated, thereby causing the tissue to vibrate, which is detected by ultrasound as a Doppler shift. For example, while nominal physiological activities and blood flowing through blood vessels may also cause a Doppler shift, the profile of Doppler shifts caused by the movement due to tissue heating from an ablation device will distinguishable from the Doppler shift caused by physiological activities and blood flow.

In addition to the Doppler effect, elastography may also be used to monitor the size, shape, and progress of the ablation zone. Elastography, as used hereinafter, is defined as an imaging modality that shows the elastic properties of tissue, and particularly soft tissue contrasted with hard tissue. Tissue that is soft will be more elastic, while tissue that is hard will be less elastic. In order to monitor the size, shape, and progress of the ablation zone using elastography, the application may, for example, receive ultrasound data from the ultrasound imaging system operating in a mode wherein the ultrasound imaging system is configured to detect elastic properties of tissue. The application may then process the received ultrasound data to identify an area where tissue is less elastic than surrounding tissue, the size and shape of such an area being indicative of an area of tissue that is undergoing ablation and the progress of the ablation procedure. Repeatedly scanning the tissue to receive new ultrasound data and processing the ultrasound data may provide updated indications of the area of tissue that is undergoing ablation and the progress of the ablation procedure. For example, as the tissue hardens due to the ablation, the ultrasound data (including ultrasound return signals) will change based on the elastic properties of the tissue, and can thus be processed to indicate the progress of the ablation procedure. The application may further cause a display device to display an indication of the size, shape, and progress of the ablation zone on ultrasound images.

The systems described herein are useful in various contexts for performing ablation treatment procedures. For example, in an embodiment in which a clinician is performing an ablation procedure, the systems may provide the clinician with various views and estimations of the size and shape of the ablation zone, as well as the progress of the ablation procedure. These and other aspects of the present disclosure are detailed herein below.

Systems for using the Doppler effect and elastography as detected by ultrasound to predict and monitor ablation zone size, shape, and progress may be implemented as part of an electromagnetic navigation (EMN) system, Generally, the EMN system may be used in planning and performing treatment of an area of the patient's lungs by determining a pathway to a target location such as a surgical site or treatment location, navigating a positioning assembly to the target location, and navigating a variety of tools to the target location. The EMN system may be configured to display various views of the patient's body, including a three-dimensional (3D) model of the patient's body generated based on pre-operative radiographic images of the patient's body.

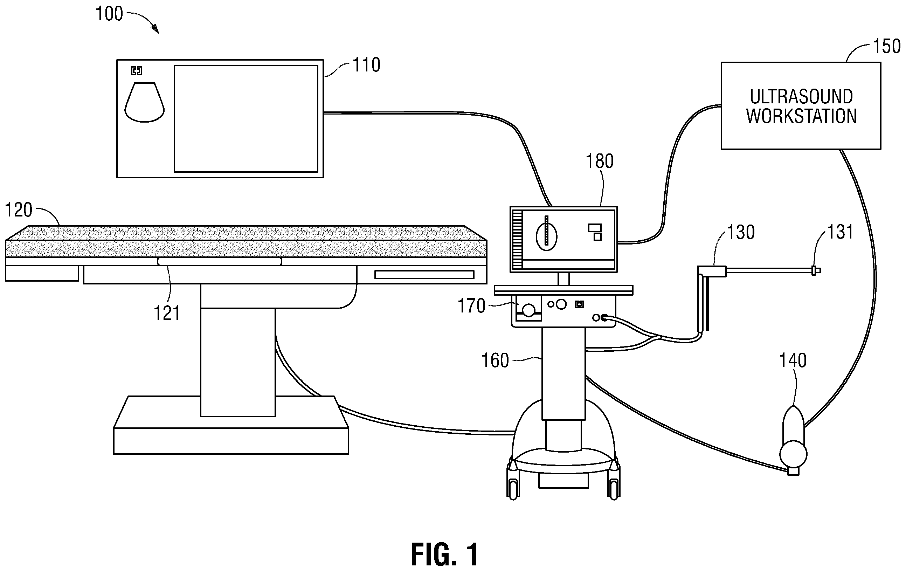

With reference to FIG. 1, a system 100 usable to predict, detect, and monitor an ablation zone using ultrasound Doppler and/or elastography detection includes a display 110, a table 120 including an electromagnetic (EM) field generator 121, a treatment tool 130 including an EM sensor 131, an ultrasound sensor 140 connected to an ultrasound workstation 150, a peristaltic pump 160, and a computing device 180 attached to or in operable communication with a microwave generator 170. Computing device 180 may be, for example, a laptop computer, desktop computer, tablet computer, or other similar device. Computing device 180 may be configured to control microwave generator 170, peristaltic pump 160, a power supply (not shown), and/or any other accessories and peripheral devices relating to, or forming part of, system 100. Display 110 is configured to output instructions, images, and messages relating to the performance of the treatment procedure.

Table 120 may be, for example, an operating table or other table suitable for use during a treatment procedure, which includes EM field generator 121. EM field generator 121 is used to generate an EM field during the treatment procedure and forms part of an EM tracking system that is used to track positions of surgical instruments within the body of a patient, such as by tracking a position of EM sensor 131. EM field generator 121 may include various components, such as a specially designed pad to be placed under, or integrated into, an operating table or patient bed. An example of such an EM tracking system is the AURORA™ system sold by Northern Digital Inc.

Treatment tool 130 is a surgical instrument for percutaneously accessing and treating a target location. For example, treatment tool 130 may be an ablation probe having a microwave ablation antenna that is used to ablate tissue. Treatment tool 130 may include, or have attached to it, EM sensor 131 enabling the EM tracking system to track the location, position, and orientation (also known as the "pose") of treatment tool 130 inside the body of the patient. Microwave generator 170 may be configured to output microwave energy to treatment tool 130 and control peristaltic pump 160. Peristaltic pump 160 may be configured to pump fluid through treatment tool 130 to cool treatment tool 130. As described in more detail below, peristaltic pump 160 may further be configured to pump fluid through treatment tool 130 in a manner to cause mechanical agitation to tissue surrounding treatment tool 130. While the present disclosure describes the use of system 100 in a surgical environment, it is also envisioned that some or all of the components of system 100 may be used in alternative settings, for example, an imaging laboratory and/or an office setting.

In addition to the EM tracking system, the surgical instruments may also be visualized by using ultrasound imaging. Ultrasound sensor 140, such as an ultrasound wand, may be used to image the patient's body during the treatment procedure to visualize the location of the surgical instruments, such as treatment tool 130, inside the patient's body. Ultrasound sensor 140 may have an EM tracking sensor embedded within or attached to the ultrasound wand, for example, a clip-on sensor or a sticker sensor. As described further below, ultrasound sensor 140 may be positioned in relation to treatment tool 130 such that treatment tool 130 is at an angle to the ultrasound image plane, thereby enabling the clinician to visualize the spatial relationship of treatment tool 130 with the ultrasound image plane and with objects being imaged. Further, the EM tracking system may also track the location of ultrasound sensor 140. In some embodiments, one or more ultrasound sensors 140 may be placed inside the body of the patient. EM tracking system may then track the location of such ultrasound sensors 140 and treatment tool 130 inside the body of the patient. Ultrasound workstation 150 may be used to configure, operate, and view images captured by ultrasound sensor 140. Likewise, computing device 180 may be used to configure, operate, and view images captured by ultrasound sensor 140, either directly or relayed via ultrasound workstation 150.

Various other surgical instruments or surgical tools, such as LIGASURE™ devices, surgical staplers, etc., may also be used during the performance of a treatment procedure. In embodiments where treatment tool 130 is a microwave ablation probe, the microwave ablation probe is used to ablate a lesion or tumor (hereinafter referred to as a "target") by using microwave energy to heat tissue in order to denature or kill cancerous cells. The construction and use of a system including such an ablation probe is more fully described in co-pending U.S. Patent Publication No. 2016/0058507, entitled MICROWAVE ABLATION SYSTEM, filed on August 26, 2014, by Dickhans, U.S. Patent No. 9,247,992, entitled MICROWAVE ABLATION CATHETER AND METHOD OF UTILIZING THE SAME, filed on March 15, 2013, by Latkow et al., and U.S. Patent No. 9,119,650, entitled MICROWAVE ENERGY-DELIVERY DEVICE AND SYSTEM, filed on March 15, 2013, by Brannan et al..

As noted above, the location of treatment tool 130 within the body of the patient may be tracked during the treatment procedure. An example of tracking the location of treatment tool 130 is by using the EM tracking system, which tracks the location of treatment tool 130 by tracking sensors, such as EM sensor 131, attached to or incorporated in treatment tool 130. Various types of sensors may be used, such as a printed sensor, the construction and use of which is more fully described in co-pending U.S. Patent Publication No. US 2016/017487314/919,950, entitled "MEDICAL INSTRUMENT WITH SENSOR FOR USE IN A SYSTEM AND METHOD FOR ELECTROMAGNETIC NAVIGATION", filed October 22, 2015, by Greenburg et al.. A percutaneous treatment system similar to the above-described system 100 is further described in co-pending U.S. Patent Application Publication No. 2016/0317224, entitled "MICROWAVE ABLATION PLANNING AND PROCEDURE SYSTEMS", filed on April 15, 2016, by Girotto et al.

While the above-described system 100 uses a microwave generator 170 to provide microwave energy to treatment tool 130, those skilled in the art will appreciate that various other types of generators and tools may be used without departing from the scope of the present disclosure. For example, radio frequency (RF) ablation tools receiving RF energy from RF generators may be substituted for the microwave generators and ablation tools described above. Further, while the above-described system 100 is designed for percutaneous access to tissue, those skilled in the art will appreciate that the approach described below may be used with systems and tools designed for endobronchial navigation to access treatment locations via the patient's airways and surrounding parenchyma without departing from the scope of the present disclosure. An example of such an endobronchial navigation system is described in U.S. Patent Application Publication No. 2016/0000302, entitled "SYSTEM AND METHOD FOR NAVIGATING WITHIN THE LUNG", filed on June 29, 2015, by Brown et al..

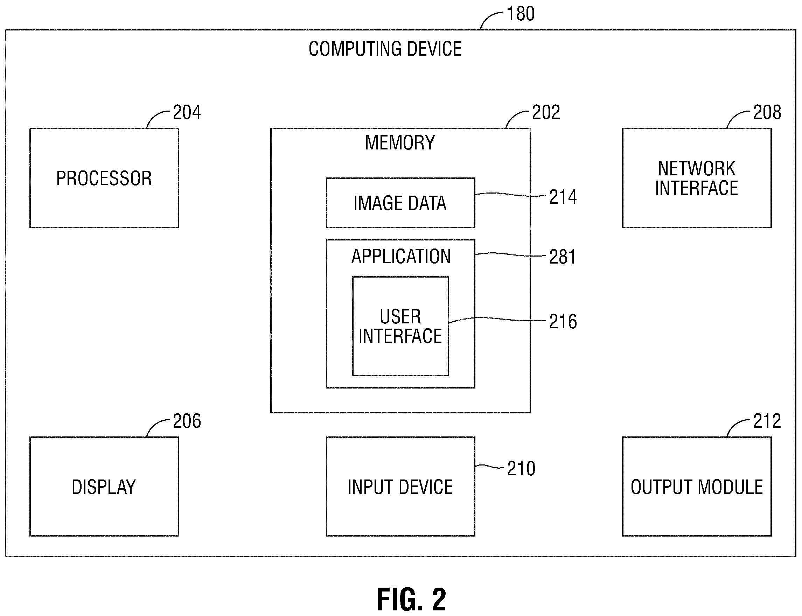

With reference to FIG. 2, there is shown a simplified block diagram of computing device 180. Computing device 180 may include at least one memory 202, one or more processors 204, a display 206, a network interface 208, one or more input devices 210, and/or an output module 212. Memory 202 may store application 281 and/or image data 214. Application 281 may, when executed by processor 204, cause display 206 to present user interface 216. Application 281 may also provide an indication of the location of treatment tool 130 in relation to the target, as well as the size, shape, and location of an ablation zone, as described further below.

Memory 202 may include any non-transitory computer-readable storage medium for storing data and/or software that is executable by processor 204 and which controls the operation of computing device 180. In an embodiment, memory 202 may include one or more solid-state storage devices such as flash memory chips. Alternatively or in addition to the one or more solid-state storage devices, memory 202 may include one or more mass storage devices connected to the processor 204 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 204. That is, computer readable storage media include non-transitory, volatile and nonvolatile, removable and non-removable media implemented in any technology for storage of information such as computer-readable instructions, data structures, program modules or other data. For example, computer-readable storage media include RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by computing device 180.

Network interface 208 may be configured to connect to a network such as a local area network (LAN) consisting of a wired network and/or a wireless network, a wide area network (WAN), a wireless mobile network, a Bluetooth network, and/or the internet. Input device 210 may be any device by means of which a user may interact with computing device 180, such as, for example, a mouse, keyboard, foot pedal, touch screen, and/or voice interface. Output module 212 may include any connectivity port or bus, such as, for example, parallel ports, serial ports, universal serial busses (USB), or any other similar connectivity port known to those skilled in the art.

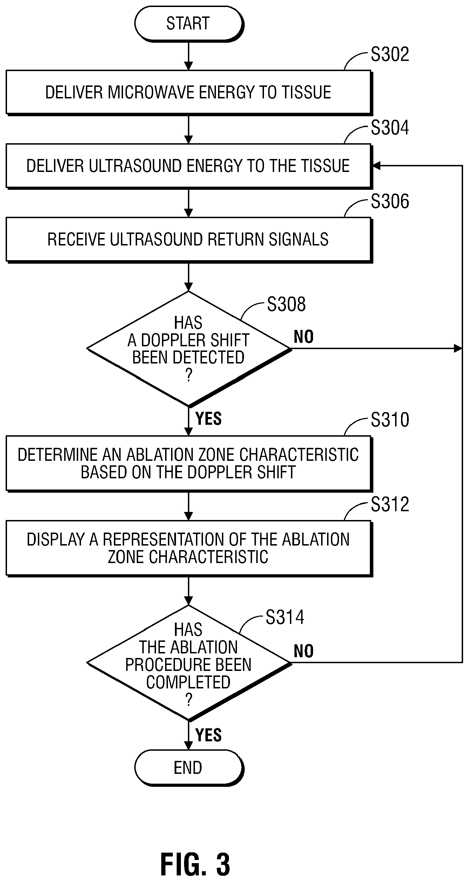

Turning now to FIG. 3, there is shown a flowchart of an exemplary method of ablation zone detection. The method may be performed using system 100 described above. In particular, application 281 executing on computing device 180 may be used to perform, or cause other components of system 100 to perform, the steps of the method of FIG. 3. Starting at step S302, microwave energy is delivered to tissue. For example, treatment tool 130 may receive microwave energy from microwave generator 170 and may deliver the microwave to the tissue upon actuation.

At step S304, ultrasound energy is delivered to the tissue. For example, ultrasound sensor 140 may be used to deliver the ultrasound energy to the tissue upon actuation. Thereafter, at step S306, ultrasound sensor 140 may receive ultrasound return signals from the tissue based on the ultrasound energy delivered to the tissue. The ultrasound return signals may then be analyzed by ultrasound workstation 150 and/or computing device 180 to determine, at step S308, whether a Doppler shift can be detected. If a Doppler shift cannot be detected, processing returns to step S304.

When a Doppler shift is detected, processing proceeds to step S310, where computing device 180 determines an ablation zone characteristic based on the Doppler shift. Computing device 180 then displays, or causes display device 110 to display, at step S312, a representation of the ablation zone characteristic. Thereafter, at step S314, application 281 determines whether the ablation procedure has been completed. If the ablation procedure has not been completed, processing returns to step S304. If the ablation procedure has been completed, processing ends.

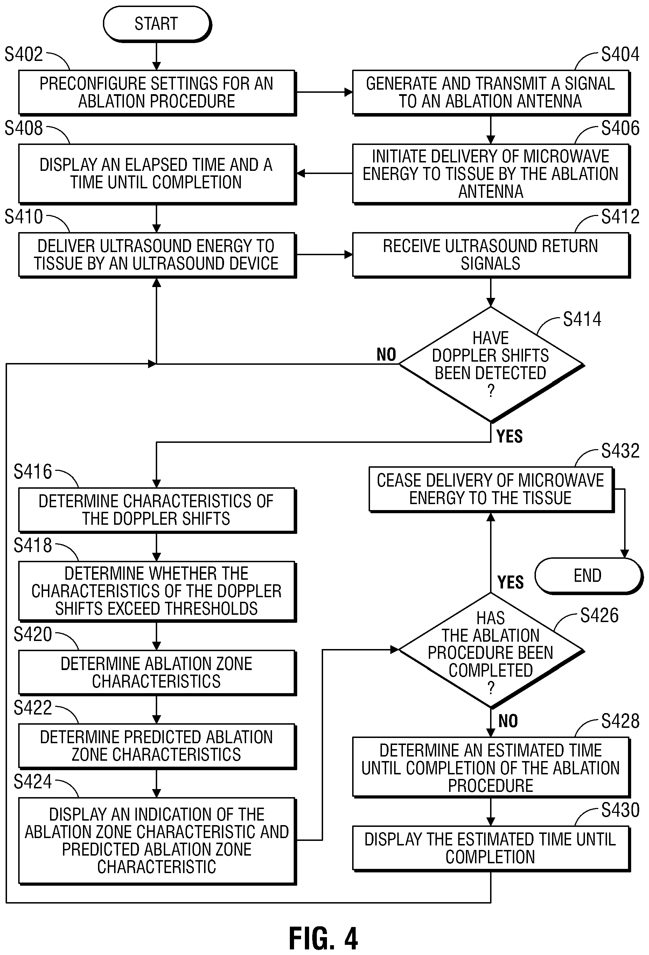

Referring now to FIG. 4, there is shown a flowchart of another exemplary method of ablation zone detection. As with the method of FIG. 3, described above, the method may be performed using system 100 described above. In particular, application 281 executing on computing device 180 may be used to perform, or cause other components of system 100 to perform, the steps of the method of FIG. 4. Starting at step S402, application 281 preconfigures settings for an ablation procedure. The settings may be received from or entered by a clinician, and/or may be stored in one or more files stored in computing device 180.

Thereafter, at step S404, application 281 generates a signal for causing an ablation antenna, such as treatment tool 130, to emit microwave energy. The signal may include an electrosurgical waveform for ablating tissue and/or instructions for emitting the electrosurgical waveform. The signal may further include an amount of power, an amplitude, an energy frequency, a frequency of pulsing, and/or a width of pulsing. Application 281 then transmits the signal to the ablation antenna. At some point prior to performing step S406, the ablation antenna is placed proximate the treatment location, as described above with reference to FIG. 1.

At step S406, the ablation antenna initiates delivery of microwave energy into tissue surrounding the ablation antenna. The delivery of microwave energy may be initiated by the clinician pressing and/or holding a button or lever on the microwave antenna, and/or by pressing a button on user interface 216 of application 281. Delivery of microwave energy into the tissue heats the tissue, thereby causing the tissue to vibrate.

Concurrent with the initiation of the delivery of microwave energy by the ablation antenna, application 281 starts a timer to record and measure the total time that has elapsed since delivery of microwave energy was started. Application 281 may then, at step S408, display the elapsed time and/or a time until completion of the ablation procedure, calculated based on the settings for the ablation procedure preconfigured at step S402, via user interface 216. The time until completion of the ablation procedure may be based on the preconfigured settings for the ablation procedure, such as a total time for the ablation procedure minus the elapsed time.

Thereafter, or concurrent therewith, at step S410, ultrasound energy is delivered to tissue at the treatment location by ultrasound sensor 140. Ultrasound sensor 140 may be positioned such that the area of tissue to which ultrasound energy is delivered encompasses all or a portion of the treatment location. Ultrasound sensor 140 then receives, at step S412, ultrasound return signals based on the ultrasound energy that is delivered to the tissue.

The ultrasound return signals are provided to ultrasound workstation and/or computing device 180 to be processed and/or analyzed, such as by application 281, to determine, at step S414, whether a Doppler shift can be detected in the tissue surrounding the ablation antenna caused by the vibration of the tissue due to the delivery of microwave energy. For example, when ultrasound sensor 140 detects a Doppler shift, movement away from ultrasound sensor 140 may be displayed in red, and movement toward ultrasound sensor 140 may be displayed in blue. Thus, in the case of tissue vibration, ultrasound sensor 140 may detect a red and blue speckled cloud, wherein tissue moving away from ultrasound sensor 140 causes red speckles, and tissue moving toward ultrasound sensor 140 causes blue speckles. As will be appreciated by those skilled in the art, the red and blue colors mentioned here are merely for example, and any two or more colors may be used to indicate the Doppler shift without departing from the scope of this disclosure. While Doppler shifts caused by tissue vibration due to moving water molecules are detectable and identifiable by application 281, Doppler shifts may also be caused by other movements, such as blood flow, tissue vibrating due to extreme heat, etc. However, Doppler shifts caused by such other movements may have a different frequency and/or intensity, and therefore are distinguishable from Doppler shifts caused by tissue heating. For the purpose of this disclosure, the Doppler shifts referred to hereinafter are Doppler shifts caused by tissue vibration due to tissue heating, as distinguished from Doppler shifts caused by other movements.

If application 281 detects a Doppler shift in the image data, processing proceeds to step S416, where application 281 further processes ultrasound image data received from ultrasound sensor 140 to determine characteristics of the Doppler shifts in the tissue surrounding the ablation antenna. The characteristics of the Doppler shifts may include an amount of Doppler shift, a rate of change of the amount of Doppler shift, a direction of Doppler shift, a rate of change of the direction of Doppler shift, a location in the tissue based upon which Doppler shift is detected, and/or a concentration of Doppler shifts detected in the location in the tissue based upon which Doppler shift is detected. For example, each Doppler shift may have a direction.

Application 281 may further determine, at step S418, whether the characteristics of the Doppler shifts exceed one or more thresholds. For example, application 281 may determine whether the amount of Doppler shift, the rate of change of the amount of the Doppler shift, and/or the concentration of Doppler shifts in a particular area exceed a threshold.

Thereafter, at step S420, application 281 determines one or more ablation zone characteristics. The determination may be based on the Doppler shift detected by ultrasound sensor 140 and/or based on the preconfigured settings of the ablation procedure. The ablation zone characteristic may further be based on a result of the determining of whether each of the plurality of characteristics exceeds the one or more thresholds. The characteristics may include a shape of an ablation zone, a size of the ablation zone, and/or a location of the ablation zone and/or one or more regions of the tissue that correspond to the Doppler shifts. Application 281 may further determine the one or more ablation zone characteristics by defining the ablation zone, including the portions of the tissue that correspond to the Doppler shifts having characteristics that exceed the one or more thresholds, and excluding the portions of the tissue that correspond to the Doppler shifts having characteristics that do not exceed the one or more thresholds, as determined at step S418.

Application 281 may, at step S422, determine predicted ablation zone characteristics. The predicted ablation zone characteristics may be based on the detected Doppler shifts, the determined ablation zone characteristics, the preconfigured configuration settings, an elapsed duration for which the microwave energy has been delivered to the tissue, an expected duration for which the microwave energy will be delivered to the tissue, a location in the tissue to which the microwave energy is delivered, and/or one or more characteristics of the signal by which the microwave energy is delivered to the tissue The predicted ablation zone characteristics may include a predicted size, shape, and/or location of the final ablation zone based on the preconfigured settings and the characteristics of the Doppler shifts. That is, application 281 may determine a prediction of the size, shape, and location of the total area that will be ablated if the ablation procedure continues as planned. Application 281 may further determine a percentage of completion of the ablation procedure based on the Doppler shifts, the determined ablation zone characteristic, an elapsed duration for which the microwave energy is delivered to the tissue, a location in the tissue to which the microwave energy is delivered, and/or one or more characteristics of a signal by which the microwave energy is delivered.

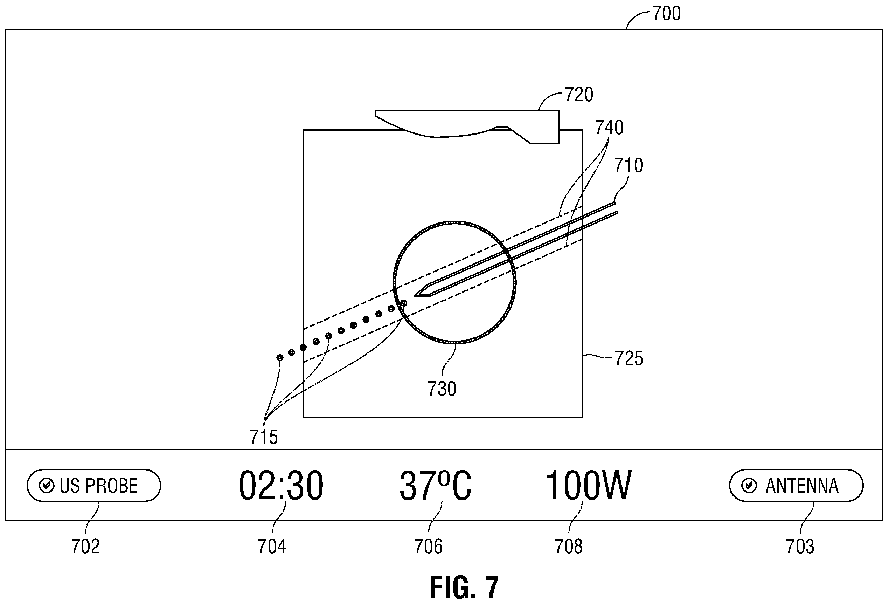

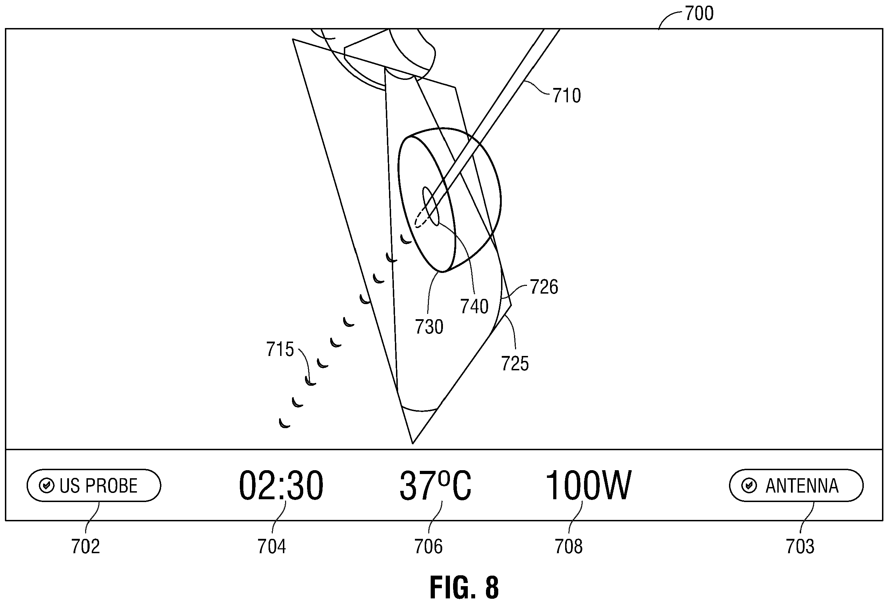

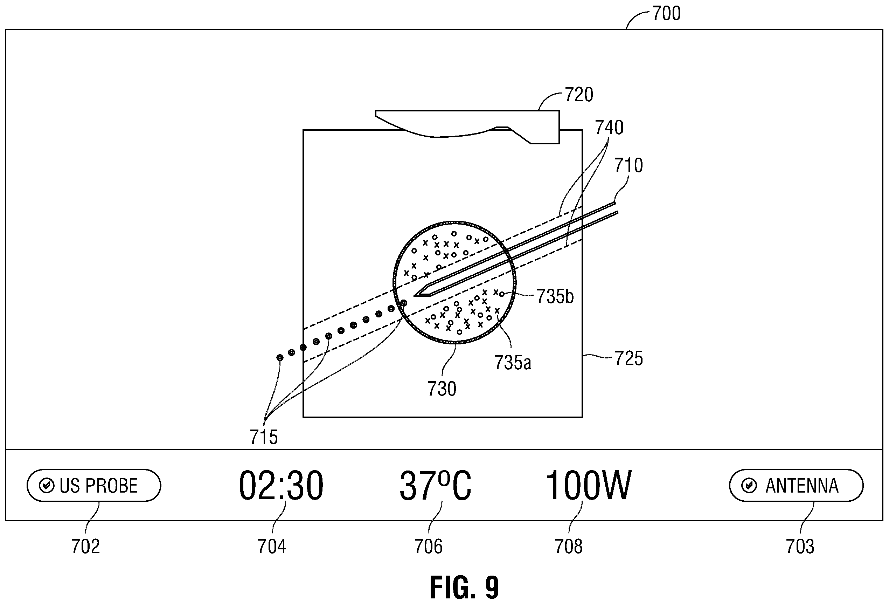

Application 281 may then, at step S424, display an indication of the ablation zone characteristics and/or the predicted ablation zone characteristics via user interface 216, examples of which are described below with reference to FIGS. 7-9. For example, application 281 may display a virtual indicator or representation of the ablation zone on ultrasound images received from ultrasound sensor 140, as shown in FIGS. 7 and 8, described below. Application 281 may further display the indication of the ablation zone as a red and blue speckled cloud, similar to the appearance of the Doppler shift, as shown in FIG. 9, described below. The virtual indicator or representation of the ablation zone characteristic may include a uniformly-colored or uniformly-shaded shape superimposed upon a portion of the ultrasound image that corresponds to an ablation zone. Alternatively, the virtual indicator or representation of the ablation zone characteristic may include a plurality of levels of shading or coloring corresponding to various levels of a characteristic of the of Doppler shifts. Application 281 may further display an indication of the percentage of completion of the ablation procedure.

Thereafter, at step S426, application 281 determines whether the ablation procedure has been completed. The determination may be based on the Doppler shifts, the determined ablation zone characteristic, an elapsed duration for which the microwave energy is delivered to the tissue, a location in the tissue to which the microwave energy is delivered, or one or more characteristics of a signal by which the microwave energy is delivered, the settings preconfigured at step S402, and/or based on a detected stoppage in the emission of microwave energy by the ablation antenna. If application 281 determines that the ablation procedure is not complete, processing proceeds to step S428, where application 281 determines an estimated time until completion of the ablation procedure. The estimated time until completion of the ablation procedure may be based on the Doppler shifts, the determined ablation zone characteristic, an elapsed duration for which the microwave energy is delivered to the tissue, a location in the tissue to which the microwave energy is delivered, one or more characteristics of the signal by which the microwave energy is delivered to the tissue, the preconfigured settings received at step S402, and/or the predicted ablation zone characteristics determined at step S422. Application 281 may then display the estimated time until completion at step S430, where after processing returns to step S410. Application 281 may further automatically adjust, by way of one or more respective settings of the ablation device, one or more characteristics of the microwave signal by which the microwave energy is delivered to the tissue based on the percentage of completion of the ablation procedure determined at step S422. Alternatively, if application 281 determines at step S426 that the ablation procedure is complete, emission of microwave energy by the ablation antenna is stopped at step S432, and processing ends. In an embodiment, application 281 may determine that the ablation procedure has been completed by analyzing the ultrasound return signals to detect whether a region of tissue where Doppler shifts were previously detected above a threshold (indicating that the tissue is being ablated) is no longer producing Doppler shifts above the threshold (indicating that the tissue has been desiccated). The Doppler shifts, which are caused by in part by water molecules moving in the tissue being ablated due to the water molecules being rapidly heated, will be most pronounced and concentrated while the tissue is being ablated and before the tissue becomes desiccated. Once the tissue becomes desiccated, and thus the majority of the water molecules are removed, the Doppler shifts due to movement of water molecules will be less pronounced and/or less concentrated. Thus, application 281 may detect and/or determine when tissue has been desiccated by analyzing the ultrasound return signals. In embodiments where a single treatment tool 130 is used to ablate the tissue, water molecules will move outward radially from the radiating portion of treatment tool 130 as the tissue surrounding treatment tool 130 is heated. Thus, the center portion of the ablated tissue, i.e. the tissue closest to the radiating portion of treatment tool 130, will be desiccated first and will be heated to the highest temperature during the ablation procedure. This portion will expand outward radially as the ablation procedure progresses. Application 281 may determine a boundary or threshold based on the settings for the ablation procedure configured or received at step S402, and may determine that the ablation procedure has been completed once the desiccated tissue extends up to and/or beyond the boundary or threshold.

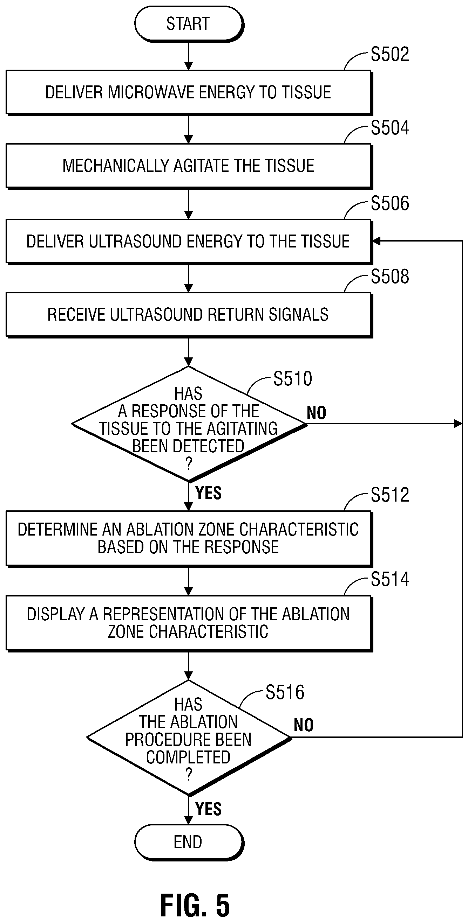

Turning now to FIG. 5, there is shown a flowchart of an exemplary method of using elastography to monitor a size and shape of an ablation zone. The method may be performed using system 100 described above. In particular, application 281 executing on computing device 180 may be used to perform, or cause other components of system 100 to perform, the steps of the method of FIG. 5. Starting at step S502, microwave energy is delivered to tissue. For example, treatment tool 130 may receive microwave energy from microwave generator 170 and may deliver the microwave to the tissue upon actuation.

At step S504, mechanical agitation is applied to the tissue. The mechanical agitation may be caused by a pulse of fluid being pumped through treatment tool 130 such as by peristaltic pump 160. The frequency and/or force with which peristaltic pump 160 pumps fluid through the ablation antenna may be modified to cause the mechanical agitation emitted by the ablation antenna to be detectable by, for example, ultrasound sensor 140.

Thereafter, at step S506, ultrasound energy is delivered to the tissue. For example, ultrasound sensor 140 may be used to deliver the ultrasound energy to the tissue upon actuation. Next, at step S508, ultrasound sensor 140 may receive ultrasound return signals from the tissue based on the ultrasound energy delivered to the tissue. The ultrasound return signals may then be analyzed by ultrasound workstation 150 and/or computing device 180 to determine, at step S510, whether a response of the tissue to the agitating can be detected. If a response of the tissue to the agitating cannot be detected, processing returns to step S504.

When a response of the tissue to the agitating is detected, processing proceeds to step S512, where computing device 180 determines an ablation zone characteristic based on the response. Computing device 180 then displays, or causes display device 110 to display, at step S514, a representation of the ablation zone characteristic. Thereafter, at step S516, application 281 determines whether the ablation procedure has been completed. If the ablation procedure has not been completed, processing returns to step S504. If the ablation procedure has been completed, processing ends.

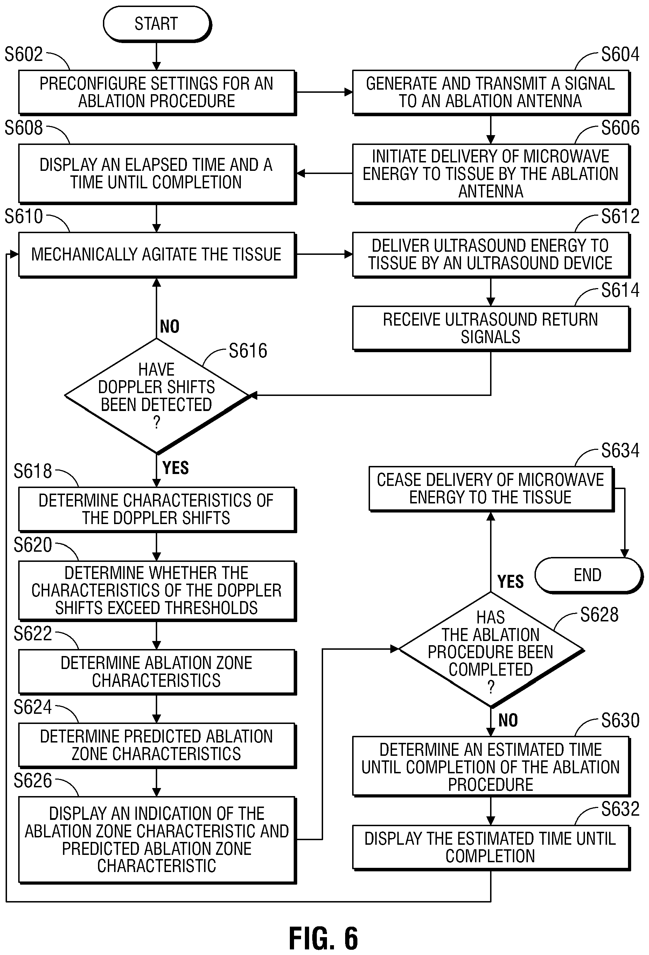

Turning now to FIG. 6, there is shown a flowchart of another exemplary method for using elastography to monitor a size and shape of an ablation zone. As with the method of FIG. 5, described above, the method may be performed using system 100 described above. In particular, application 281 executing on computing device 180 may be used to perform, or cause other components of system 100 to perform, the steps of the method of FIG. 6. Starting at step S602, application 281 preconfigures settings for an ablation procedure. The settings may be received from or entered by a clinician, and/or may be stored in one or more files stored in computing device 180.

Thereafter, at step S604, application 281 generates a signal for causing an ablation antenna, such as treatment tool 130, to emit microwave energy. The signal may include an amount of power, an amplitude, an energy frequency, a frequency of pulsing, or a width of pulsing, an electrosurgical waveform for ablating tissue, and/or instructions for delivering the electrosurgical waveform. Application 281 then transmits the signal to the ablation antenna. At some point prior to performing step S606, the ablation antenna is placed proximate the treatment location, as described above with reference to FIG. 1.

At step S606, the ablation antenna initiates delivery of microwave energy into tissue surrounding the ablation antenna. The delivery of microwave energy may be initiated by the clinician pressing and/or holding a button or lever on the microwave antenna, and/or by pressing a button on user interface 216 of application 281. Delivery of microwave energy into the tissue heats the tissue, thereby causing the tissue to stiffen or harden, thus becoming less elastic than before the delivery of microwave energy into the tissue. The tissue into which microwave energy is delivered will also become less elastic than adjacent tissue. That is, tissue located closer to the ablation antenna and thus receiving more microwave energy and being heated to a higher temperature, will be less elastic, and thus have a lower level of elasticity, than tissue located further away from the ablation antenna.

Concurrent with the initiation of the delivery of microwave energy by the ablation antenna, application 281 starts a timer to record and measure the total time that has elapsed since delivery of microwave energy was started. Application 281 may then, at step S608, display the elapsed time and/or a time until completion of the ablation procedure, calculated based on the settings for the ablation procedure preconfigured at step S602, via user interface 216. The time until completion of the ablation procedure may be based on the preconfigured settings for the ablation procedure, such as a total time for the ablation procedure minus the elapsed time.

Thereafter, or concurrent therewith, at step S610, the ablation antenna emits mechanical agitation into the tissue surrounding the ablation antenna. The mechanical agitation may be caused by a pulse of fluid being pumped through the microwave ablation antenna, such as by peristaltic pump 160. The frequency and/or force with which peristaltic pump 160 pumps fluid through the ablation antenna may be modified to cause the mechanical agitation emitted by the ablation antenna to be detectable by, for example, ultrasound return signals and/or ultrasound images captured by ultrasound sensor 140. For example, peristaltic pump 160 may be configured to produce pulses at approximately 10Hz during normal operation. The pulse frequency may be adjusted or modified, such as by speeding up or slowing down the motor head of peristaltic pump 160, to optimize the effectiveness of a pressure wave generated by the pulse. A response of the tissue to the agitating may be accentuated based upon effectiveness of the pressure wave, and a characteristic of the response of the tissue, such as an elasticity gradient, may be determined based upon the response of the tissue to the agitating. The elasticity gradient may be indicative of a level of desiccation of the tissue, and thus of the progress of the ablation procedure. Various pulse frequencies may be generated by varying the motor head speeds of peristaltic pump 160, and thus may accentuate an elasticity gradient at different distances from the ablation antenna. For example, a higher pulse frequency may accentuate an elasticity gradient closer to the ablation antenna, while a lower frequency may accentuate an elasticity gradient further from the ablation antenna. An increased pulse frequency will diminish the pressure spike or force of the pulses, thereby causing the elasticity gradient to be accentuated closer to the ablation antenna, while a reduced pulse frequency will increase the pressure spike or force of the pulses, thereby causing the elasticity gradient to be accentuated further from the ablation antenna.

Next, at step S612, ultrasound energy is delivered to tissue at the treatment location by ultrasound sensor 140. Ultrasound sensor 140 may be positioned such that the area of tissue to which ultrasound energy is delivered encompasses all or a portion of the treatment location. Ultrasound sensor 140 then receives, at step S614, ultrasound return signals based on the ultrasound energy that is delivered to the tissue.

The ultrasound return signals are provided to ultrasound workstation and/or computing device 180 to be processed and/or analyzed, such as by application 281, to determine, at step S616, whether a response to the agitating can be detected in the tissue surrounding the ablation antenna. For example, as described above, the mechanical agitation may displace tissue surrounding the ablation antenna based on a pulse of fluid through the ablation antenna. In embodiments, application 281 may adjust a frequency and/or a force with which peristaltic pump 160 pumps fluid through the ablation device to cause mechanical agitation to the tissue surrounding the ablation deice, and the response of the tissue to the agitating may be detected based on the adjusted frequency and/or force. If application 281 determines that a response to the agitating has not been detected, processing returns to step S610.

Alternatively, if a response to the agitating has been detected, processing proceeds to step S618 where application 281 processes the ultrasound return signals and/or ultrasound images to determine characteristics of the response of the tissue to the agitating. The characteristics of the response of the tissue to the agitating may include an amount of displacement of the tissue, a rate of change of the amount of displacement of the tissue, a direction of displacement of the tissue, a rate of change of the direction of displacement of the tissue, a location in the tissue based upon which displacement of the tissue is detected, an amount of time the tissue takes to return to a pre-agitated position, and/or an elasticity gradient. That is, application 281 may determine, based on the detected displacement of the tissue surrounding the ablation antenna caused by the mechanical agitation, a reaction time of the tissue, i.e., a time it takes the tissue to return to its pre-agitated state. For example, the ultrasound images may show that the tissue surrounding the ablation antenna is displaced and returned to its pre-displaced position every time a pulse of fluid is pumped through the ablation antenna. Application 281 may also determine an amount of displacement of the tissue based on the mechanical agitation. An elasticity gradient may be based on one or more of these characteristics. The amount of displacement and the reaction time of the tissue will be correlated with the level of elasticity of the tissue. Tissue that is less elastic will move less and have a quicker reaction time than tissue that is more elastic. As noted above, tissue that is less elastic will have received more microwave energy and have been heated to a higher temperature than tissue that is more elastic, and therefore an inference can be made that tissue that moves less and has a quicker reaction time has been more thoroughly ablated than tissue that moves more.

In embodiments, application 281 may perform differential analysis of the response of the tissue to the agitating. For example, application 281 may analyze the response of the tissue to the agitating across a scanning plane of ultrasound sensor 140 to focus on a difference in elasticity or stiffness between neighboring tissue regions. If application 281 detects that a tissue region is displaced more or less than a neighboring tissue region, application 281 may identify this differential as a gradient line and/or a potential ablation zone boundary. If a similar differential is detected along a consistent line or at a consistent distance from the ablation antenna, application 281 may improve a confidence level that the identified gradient line is the ablation zone boundary. Similarly, application 281 may identify boundaries of the tumor based on differential analysis of gradient lines based on the response of the tissue to the agitating. For example, cancerous tissue may have a different response to agitation than healthy tissue, and the difference in the response of the cancerous tissue may be detectable based on differential analysis of the gradient lines. Thus, in embodiments, differential analysis of the response of the tissue to the agitating may be performed after the ablation antenna has been placed but before the initiation of the delivery of microwave energy to the tissue, e.g. prior to step S606, to identify the boundaries of the tumor. In such embodiments, steps S610 and S612 may be performed prior to step S606.

Thereafter, at step S620, application 281 compares the characteristics of the response of the tissue with one or more predetermined thresholds to determine if each of the characteristics exceeds the one or more thresholds. For example, if the reaction time is less than the predetermined threshold, that may be an indication that the tissue is less elastic. Alternatively, if the reaction time is greater than the predetermined threshold, that may be an indication that the tissue is more elastic.

Application 281 may then, at step S622, determine characteristics of an ablation zone surrounding the ablation antenna based on the characteristics of the response of the tissue. For example, the determination may be based on the level of elasticity, amount of displacement, and/or reaction time of the tissue detected by ultrasound sensor 140. Alternatively or in addition, the determination may be based on gradient lines detected based on the differential analysis, described above. The determination may further be based on a region of the tissue that corresponds to the detected response of the tissue to the agitating, and/or the result of the determination at step S620 of whether the characteristics of the response of the tissue exceed the one or more thresholds. The ablation zone characteristics may include a shape of the ablation zone, a size of the ablation zone, and/or a location of the ablation zone. Determining the ablation zone characteristic may further include defining the ablation zone by including the portions of the tissue that correspond to the response of the tissue having characteristics that exceed the one or more thresholds, and excluding the portions of the tissue that correspond to the response of the tissue having characteristics that do not exceed the one or more thresholds.

Application 281 may further determine, at step S624, predicted ablation zone characteristics. The predicted ablation zone characteristics may include a predicted size, shape, and/or location of the final ablation zone based on the preconfigured settings and the characteristics of the response of the tissue to the agitating. That is, application 281 may determine a prediction of the size, shape, and location of the total area that will be ablated if the ablation procedure continues as planned. The predicted ablation zone characteristic may be based on the response of the tissue, the determined ablation zone characteristic, a setting of the ablation device, an elapsed duration for which the microwave energy is delivered to the tissue, an expected duration for which the microwave energy will be delivered to the tissue, a location in the tissue to which the microwave energy is delivered, or one or more characteristics of the signal by which the microwave energy is delivered to the tissue. Application 281 may further determine a percentage of completion of the ablation procedure based on the response of the tissue, the determined ablation zone characteristic, an elapsed duration for which the microwave energy is delivered to the tissue, a location in the tissue to which the microwave energy is delivered, and/or one or more characteristics of a signal by which the microwave energy is delivered.

Application 281 may then, at step S626, display an indication of the ablation zone characteristics and/or the predicted ablation zone characteristics via user interface 216, examples of which are described below with reference to FIGS. 7 and 8. For example, application 281 may display a virtual indicator or representation of the ablation zone as an overlay on ultrasound images received from ultrasound sensor 140, as shown in FIGS. 7 and 8, described below. The virtual indicator or representation of the ablation zone characteristic may include a uniformly-colored or uniformly-shaded shape superimposed upon a portion of the ultrasound image that corresponds to an ablation zone. Alternatively, the virtual indicator or representation of the ablation zone characteristic may include a plurality of levels of shading or coloring corresponding to various levels of a characteristic of the of response of the tissue. Application 281 may further display the indication of the ablation zone as a gradient indicating different levels of elasticity of the tissue, with tissue closer to the ablation antenna being less elastic and tissue further from the ablation antenna being more elastic. The virtual indicator or representation of the ablation zone may further include a shape having a plurality of levels of shading or coloring corresponding to a plurality of respective levels of a characteristic of the response of the tissue. Application 281 may further display an indication of the percentage of completion of the ablation procedure. Additionally, application 281 may display the gradient lines identified during the differential analysis, described above, where the gradient lines may be indicative of a boundary of the ablation zone and/or a boundary of the tumor.

The progress of the ablation procedure may be tracked based on repeated emissions of mechanical agitation into the tissue to repeatedly measure the displacement and reaction time of the tissue, as detected at step S616, and the determined characteristics of the ablation zone determined at step S622. For example, application 281 may determine an area of tissue that has been sufficiently ablated, based on the detected level of elasticity and reaction time of the tissue, and/or an area of tissue that has not yet been sufficiently ablated. Application 281 may then display an indicator of the different areas and their corresponding levels of elasticity, and whether or not those areas have been sufficiently ablated, on the ultrasound images received from ultrasound sensor 140.

Thereafter, at step S628, application 281 determines whether the ablation procedure has been completed. The determination may be based on the response of the tissue, the determined ablation zone characteristic, an elapsed duration for which the microwave energy is delivered to the tissue, a location in the tissue to which the microwave energy is delivered, one or more characteristics of the signal by which the microwave energy is delivered, the settings preconfigured at step S602, and/or based on a detected stoppage in the emission of microwave energy by the ablation antenna.

If application 281 determines that the ablation procedure is not complete, processing proceeds to step S630, where application 281 determines an estimated time until completion of the ablation procedure. The estimated time until completion of the ablation procedure may be based on the response of the tissue, the determined ablation zone characteristic, an elapsed duration for which the microwave energy is delivered to the tissue, a location in the tissue to which the microwave energy is delivered, or one or more characteristics of the signal by which the microwave energy is delivered, the preconfigured settings received at step S602, and/or the predicted ablation zone characteristics determined at step S624. Application 281 may then display the estimated time until completion at step S632, where after processing returns to step S610. Application 281 may further automatically adjust, by way of one or more respective settings of the ablation device, one or more characteristics of the microwave signal by which the microwave energy is delivered to the tissue based on the percentage of completion of the ablation procedure determined at step S624.

Alternatively, if application 281 determines at step S628 that the ablation procedure is complete, emission of microwave energy by the ablation antenna is stopped at step S634, and processing ends.

As will be appreciated by those skilled in the art, the methods described above are subroutines forming part of a lengthier process for performing an ablation procedure. Therefore, various other steps and routines that form part of an ablation procedure, as is known by those skilled in the art, are omitted or only briefly described herein for purposes of brevity.

FIG. 7 shows a diagram of an exemplary user interface 216 that may be displayed by application 281 on display 206 during step S424 of FIG. 4 and/or step S626 of FIG. 6, as described above. FIG. 7 includes a view 700 showing an indicator for an ultrasound probe 702 and an indicator for an ablation antenna 703. Also shown are a time 704, a temperature 706, and an output wattage 708. Time 704 may be configured to display elapsed time and/or a remaining time, as described above with reference to FIGS. 4 and 6. View 700 also shows a representation of an ablation antenna 710 overlaid onto an ultrasound image plane 725. Ablation antenna 710 may include one or more parts, for example, an imaged part corresponding to treatment tool 130 as detected in the ultrasound image data, and a generated part corresponding to one or more portions of treatment tool 130 that are not detected in the ultrasound image data and rather displayed as a graphical overlay onto the ultrasound image data. An ultrasound sensor 720 is shown to provide perspective of the angle of ultrasound image plane 725. An angle of intersection indicator 740 is shown to provide perspective of the angle of intersection of ablation antenna 710 with ultrasound image plane 725. As shown in FIG. 7, ablation antenna 710 and ultrasound image plane 725 are co-planar. A trajectory 715 of ablation antenna 710 may also be shown. An indicator of a projected and/or detected ablation zone 730 is shown around ablation antenna 710. The indicator of the projected and/or detected ablation zone 730 corresponds to the indicator of the ablation zone described above with reference to FIGS. 4 and 6.

Turning now to FIG. 8, there is shown another exemplary user interface 216 that may be displayed by application 281 on display 206 during step S424 of FIG. 4 and/or step S626 of FIG. 6, as described above. FIG. 8 includes many of the same elements as FIG. 7, described above. Such elements will not be described again for purpose of brevity. FIG. 8 shows a pseudo-3D perspective view of ultrasound image plane 725 and the angle of intersection with ablation antenna 710. Ultrasound images 726 are displayed on ultrasound image plane 725, while ablation antenna 710 is shown intersecting ultrasound image plane 725 at an angle represented by angle of intersection indicator 740.

Referring now to FIG. 9, there is shown yet another exemplary user interface 216 that may be displayed by application 281 on display 206 during step S424 of FIG. 4, described above. FIG. 9 includes many of the same elements as FIG. 7, described above. Such elements will not be described again for purpose of brevity. FIG. 9 shows an optional view similar to FIG. 7 wherein a red and blue speckled cloud is shown about the ablation zone. As described above, the speckles may be indicated by various colors, shapes, and/or other indicators. As shown in FIG. 9, red speckles are represented by "x" markers 735a, and blue speckles are represented by "o" markers 735b. During the course of the ablation procedure, the red and blue speckles may be presented about the ablation zone in locations where Doppler shifts are detected. As the ablation procedure progresses, as described above, the tissue regions closest to the ablation antenna will become desiccated first, and Doppler shifts in such tissue regions may decrease in amount, concentration, and/or frequency, As such, by viewing a user interface 216 such as shown in FIG. 9, the clinician may visualize the progress of the ablation procedure based on the tissue regions where Doppler shifts are presented,

Detailed embodiments of devices, and systems incorporating such devices, are described herein. However, these detailed embodiments are merely examples of the disclosure, which may be embodied in various forms. Therefore, specific structural and functional details disclosed herein are not to be interpreted as limiting, but merely as a basis for the claims and as a representative basis for allowing one skilled in the art to variously employ the present disclosure in appropriately detailed structure.

## Claims

1. A system (100) for ablation zone detection, the system comprising:
an ablation device (130) configured to deliver microwave energy to tissue at a treatment location;
a computing device (180) including a processor (204) and a memory (202) storing instructions which, when executed by the processor, cause the computing device to:
receive a plurality of ultrasound return signals from an ultrasound device (140), wherein the ultrasound device is configured to deliver ultrasound energy to tissue and receive, from a plurality of respective portions of the tissue, the plurality of ultrasound return signals that are based on the delivered ultrasound energy,
detect, in the plurality of ultrasound return signals, a plurality of respective Doppler shifts that are based on the microwave energy delivered to the tissue,
determine an ablation zone characteristic based on the plurality of Doppler shifts, and
cause a display device (206) to display, by way of a graphical user interface (216), an image representing at least a portion of the tissue and a representation of the ablation zone characteristic,
the computing device being further configured to display on the display device, based on the plurality of Doppler shifts, an indication of the size and shape of the ablation zone (730) on an ultrasound image.

2. The system according to claim 1, wherein the plurality of Doppler shifts have a plurality of directions, and wherein the determining the ablation zone characteristic includes identifying one or more regions of the tissue that correspond to one or more of the plurality of Doppler shifts, respectively.

3. The system according to claim 1, wherein the instructions, when executed by the processor, further cause the computing device to:
determine a plurality of characteristics of the plurality of Doppler shifts,
wherein the plurality of characteristics includes any one or a combination of an amount of Doppler shift, a rate of change of the amount of Doppler shift, a direction of Doppler shift, a rate of change of the direction of Doppler shift, or a location in the tissue based upon which Doppler shift is detected; and
determine whether each of the plurality of characteristics exceeds one or more thresholds,
wherein the determining of the ablation zone characteristic is further based on a result of the determining of whether each of the plurality of characteristics exceeds the one or more thresholds.

4. The system according to claim 3, wherein the determining of the ablation zone characteristic includes defining an ablation zone,
the portions of the tissue that correspond to the plurality of Doppler shifts having respective characteristics that exceed the one or more thresholds being included in the ablation zone, and
the portions of the tissue that correspond to the plurality of Doppler shifts having respective characteristics that do not exceed the one or more thresholds being excluded from the ablation zone,
wherein the ablation zone characteristic includes a combination of the shape, size, and location of the ablation zone.

5. The system according to claim 1, wherein the instructions, when executed by the processor, further cause the computing device to:
determine a predicted ablation zone characteristic, if the ablation continues as planned, based on any one or a combination of the plurality of Doppler shifts, the determined ablation zone characteristic, a setting of the ablation device, an elapsed duration for which the microwave energy is delivered to the tissue, an expected duration for which the microwave energy will be delivered to the tissue, a location in the tissue to which the microwave energy is delivered, or one or more characteristics of a signal by which the microwave energy is delivered to the tissue; and
cause the display device to display, by way of the graphical user interface, a representation of the predicted ablation zone characteristic.

6. The system according to claim 5, wherein the one or more characteristics of the signal by which the microwave energy is delivered include any one or a combination of an amount of power of the signal, an amplitude of the signal, a frequency of the signal, a frequency of pulsing of the signal, or a width of pulsing of the signal.

7. The system according to claim 1, wherein the instructions, when executed by the processor, further cause the computing device to:
determine an estimated amount of time until completion of an ablation procedure, based on any one or a combination of the plurality of Doppler shifts, the determined ablation zone characteristic, an elapsed duration for which the microwave energy is delivered to the tissue, a location in the tissue to which the microwave energy is delivered, or one or more characteristics of a signal by which the microwave energy is delivered; and
cause the display device to display, via the graphical user interface, an indicator of the estimated amount of time until completion of the ablation procedure.

8. The system according to claim 1, wherein the instructions, when executed by the processor, further cause the computing device to:
determine whether an ablation procedure is completed based on any one or a combination of the plurality of Doppler shifts, the determined ablation zone characteristic, an elapsed duration for which the microwave energy is delivered to the tissue, or a location in the tissue to which the microwave energy is delivered, or one or more characteristics of a signal by which the microwave energy is delivered; and
cause the display device to display, via the graphical user interface, an indicator of whether the ablation procedure is completed.

9. The system according to claim 8, wherein the instructions, when executed by the processor, further cause the computing device to:
automatically disable the delivering of the microwave energy to the tissue in response to a determination that the ablation procedure is completed.

10. The system according to claim 1, wherein the instructions, when executed by the processor, further cause the computing device to:
determine a percentage of completion of an ablation procedure based on any one or a combination of the plurality of Doppler shifts, the determined ablation zone characteristic, an elapsed duration for which the microwave energy is delivered to the tissue, a location in the tissue to which the microwave energy is delivered, or one or more characteristics of a signal by which the microwave energy is delivered; and
cause the display device to display, via the graphical user interface, an indicator of the percentage of completion of the ablation procedure.

11. The system according to claim 1, wherein the microwave energy causes the tissue to vibrate, and wherein the instructions, when executed by the processor, cause the computing device to process the received ultrasound return signals to identify an area where tissue is vibrating and thus causing a red-and-blue speckled cloud detectable in the ultrasound return signals as a Doppler shift, the red-and-blue speckled cloud being indicative of the size and shape of the ablation zone.

## Patentansprüche

1. System (100) zur Erkennung von Ablationszonen, wobei das System Folgendes umfasst:
eine Ablationsvorrichtung (130), die konfiguriert ist, um dem Gewebe an einer Behandlungsstelle Mikrowellenenergie zuzuführen;
eine Rechenvorrichtung (180) mit einem Prozessor (204) und einem Speicher (202), der Befehle speichert, die bei ihrer Ausführung durch den Prozessor die Rechenvorrichtung zu Folgendem veranlassen:
Empfangen der mehreren Ultraschall-Echosignale von einem Ultraschallgerät (140), wobei das Ultraschallgerät konfiguriert ist, um dem Gewebe Ultraschallenergie zuzuführen und von mehreren entsprechenden Teilen des Gewebes mehrere Ultraschall-Echosignale zu empfangen, die auf der abgegebenen Ultraschallenergie basieren,
Erfassen, in den mehreren Ultraschall-Echosignalen, mehrerer entsprechender Doppler-Verschiebungen, die auf der an das Gewebe abgegebenen Mikrowellenenergie basieren,
Ermitteln eines Ablationszonenmerkmals auf der Grundlage der mehreren Doppler-Verschiebungen, und
die ein Anzeigegerät (206) dazu veranlassen, mittels einer graphischen Benutzeroberfläche (216) ein Bild anzuzeigen, das mindestens einen Teil des Gewebes und eine Darstellung des Ablationszonenmerkmals darstellt,
wobei die Rechenvorrichtung ferner so konfiguriert ist, dass sie auf der Anzeigevorrichtung, basierend auf den mehreren Doppler-Verschiebungen, eine
Angabe von Größe und Form der Ablationszone (730) auf einem Ultraschallbild anzeigt.

2. System nach Anspruch 1, wobei die mehreren Doppler-Verschiebungen mehrere Richtungen haben und wobei das Ermitteln des Ablationszonenmerkmals das Identifizieren einer oder mehrerer Zonen des Gewebes einschließt, die jeweils einer oder mehreren der mehreren Doppler-Verschiebungen entsprechen.

3. System nach Anspruch 1, wobei die Befehle bei ihrer Ausführung durch den Prozessor die Rechenvorrichtung ferner zu Folgendem veranlassen:
Ermitteln mehrerer Merkmale der mehreren Doppler-Verschiebungen,
wobei die mehreren Merkmale eine beliebige aus einer Kombination aus einem Wert der Doppler-Verschiebung, einer Änderungsrate des Wertes der Doppler-Verschiebung, einer Richtung der Doppler-Verschiebung, einer Änderungsrate der Richtung der Doppler-Verschiebung oder einer Stelle im Gewebe, auf deren Grundlage die Doppler-Verschiebung erfasst wird, umfassen; und
Ermitteln, ob jedes der mehreren Merkmale eine oder mehrere Schwellenwerte überschreitet, wobei das Ermitteln des Ablationszonenmerkmals ferner auf einem Ergebnis der Ermittlung, ob jedes der mehreren Merkmale, die eine oder die mehreren Schwellenwerte überschreitet, basiert.

4. System nach Anspruch 3, wobei das Ermitteln des Ablationszonenmerkmals das Definieren einer Ablationszone umfasst,
wobei die Teile des Gewebes, die den mehreren Doppler-Verschiebungen entsprechen, entsprechende Merkmale aufweisen, die den einen oder die mehreren Schwellenwerte überschreiten, die in der Ablationszone enthalten sind, und
wobei die Teile des Gewebes, die den mehreren Doppler-Verschiebungen entsprechen, entsprechende Merkmale aufweisen, die die eine oder die mehreren Schwellenwerte nicht überschreiten, die von der Ablationszone ausgeschlossen sind,
wobei das Merkmal der Ablationszone eine Kombination aus der Form, Größe und Lage der Ablationszone umfasst.

5. System nach Anspruch 1, wobei die Befehle bei ihrer Ausführung durch den Prozessor die Rechenvorrichtung ferner zu Folgendem veranlassen:
Ermitteln eines prognostizierten Ablationszonenmerkmals, wenn die Ablation wie geplant fortgesetzt wird, auf der Grundlage einer oder einer Kombination der mehreren Doppler-Verschiebungen, des ermittelten Ablationszonenmerkmals; einer Einstellung der Ablationsvorrichtung, einer verstrichenen Dauer, für die die Mikrowellenenergie an das Gewebe abgegeben wird, einer erwarteten Dauer, für die die Mikrowellenenergie an das Gewebe abgegeben wird, einer Stelle im Gewebe, an die die Mikrowellenenergie abgegeben wird, oder eines oder mehrerer Merkmale eines Signals, durch das die Mikrowellenenergie an das Gewebe abgegeben wird; und wobei sie
das Anzeigegerät dazu veranlassen, über die graphische Benutzeroberfläche eine Darstellung des prognostizierten Ablationszonenmerkmals darzustellen.

6. System nach Anspruch 5, wobei das eine oder die mehreren Merkmale des Signals, durch das die Mikrowellenenergie übertragen wird, irgendeine oder eine Kombination aus einem Leistungswert des Signals, einer Amplitude des Signals, einer Frequenz des Signals, einer Impulsfrequenz des Signals oder einer Impulsbreite des Signals umfassen.

7. System nach Anspruch 1, wobei die Befehle bei ihrer Ausführung durch den Prozessor die Rechenvorrichtung ferner zu Folgendem veranlassen:
Ermitteln einer geschätzten Zeitdauer bis zum Abschluss eines Ablationsverfahrens auf der Grundlage einer oder einer Kombination aus den mehreren Doppler-Verschiebungen, des ermittelten Ablationszonenmerkmals, einer verstrichenen Dauer, für die die Mikrowellenenergie an das Gewebe abgegeben wird, einer Stelle im Gewebe, an die die Mikrowellenenergie abgegeben wird, oder eines oder mehrerer Merkmale eines Signals, durch das die Mikrowellenenergie abgegeben wird; und
das Anzeigegerät dazu veranlassen, über die graphische Benutzeroberfläche einen Indikator für die geschätzte Zeit bis zum Abschluss des Ablationsvorgangs anzuzeigen.

8. System nach Anspruch 1, wobei die Befehle bei ihrer Ausführung durch den Prozessor die Rechenvorrichtung ferner zu Folgendem veranlassen:
Ermitteln, ob ein Ablationsverfahren abgeschlossen ist, auf der Grundlage einer oder einer Kombination der mehreren Doppler-Verschiebungen, des ermittelten Ablationszonenmerkmals, einer verstrichenen Dauer, für die die Mikrowellenenergie an das Gewebe abgegeben wird, oder einer Stelle im Gewebe, an die die Mikrowellenenergie abgegeben wird, oder eines oder mehrerer Merkmale eines Signals, durch das die Mikrowellenenergie abgegeben wird; und
das Anzeigegerät dazu veranlassen, über die graphische Benutzeroberfläche einen Indikator anzuzeigen, ob der Ablationsvorgang abgeschlossen ist.

9. System nach Anspruch 8, wobei die Befehle bei ihrer Ausführung durch den Prozessor die Rechenvorrichtung ferner zu Folgendem veranlassen:
automatischem Deaktivieren der Mikrowellenabgabe an das Gewebe als Reaktion auf die Feststellung, dass der Ablationsvorgang abgeschlossen ist.

10. System nach Anspruch 1, wobei die Befehle bei ihrer Ausführung durch den Prozessor die Rechenvorrichtung ferner zu Folgendem veranlassen:
Ermitteln eines prozentualen Abschlussfortschritts eines Ablationsverfahrens auf der Grundlage einer oder einer Kombination aus den mehreren Doppler-Verschiebungen, des ermittelten Ablationszonenmerkmals, einer verstrichenen Dauer, für die die Mikrowellenenergie an das Gewebe abgegeben wird, einer Stelle im Gewebe, an die die Mikrowellenenergie abgegeben wird, oder eines oder mehrerer Merkmale eines Signals, durch das die Mikrowellenenergie abgegeben wird; und
das Anzeigegerät dazu veranlassen, über die graphische Benutzeroberfläche einen Indikator für den prozentualen Abschlussfortschritt des Ablationsvorgangs anzuzeigen.

11. System nach Anspruch 1, wobei die Mikrowellenenergie das Gewebe zum Schwingen bringt und wobei die Befehle bei ihrer Ausführung durch den Prozessor die Rechenvorrichtung dazu veranlassen, die empfangenen Ultraschall-Echosignale zu verarbeiten, um einen Bereich zu identifizieren, in dem das Gewebe schwingt und somit eine rot-blau gesprenkelte Wolke verursacht, die in den Ultraschall-Echosignalen als Doppler-Verschiebung erkennbar ist, wobei die rot-blau gesprenkelte Wolke die Größe und Form der Ablationszone anzeigt.

## Revendications

1. Système (100) pour la détection de zone d'ablation, le système comprenant :
un dispositif d'ablation (130) conçu pour distribuer une énergie micro-onde aux tissus à un emplacement de traitement ;
un dispositif informatique (180) comportant un processeur (204) et une mémoire (202) stockant des instructions qui, lorsqu'elles sont exécutées par le processeur, amènent le dispositif informatique à :
recevoir la pluralité de signaux de retour d'ultrasons d'un dispositif d'ultrasons (140), le dispositif d'ultrasons étant conçu pour distribuer de l'énergie ultrasonore aux tissus et recevoir, à partir d'une pluralité de parties respectives du tissu, une pluralité de signaux de retour d'ultrasons qui sont basés sur l'énergie ultrasonore distribuée,
détecter, dans la pluralité de signaux de retour d'ultrasons, une pluralité de décalages Doppler respectifs qui sont basés sur l'énergie micro-onde distribuée au tissu,
déterminer une caractéristique de zone d'ablation sur la base de la pluralité de décalages Doppler, et à
amener un dispositif d'affichage (206) à afficher, au moyen d'une interface utilisateur graphique (216), une image représentant au moins une partie du tissu et une représentation de la caractéristique de la zone d'ablation,
le dispositif informatique étant en outre conçu pour afficher sur le dispositif d'affichage, sur la base de la pluralité de décalages Doppler, une
indication de la taille et de la forme de la zone d'ablation (730) sur une image ultrasonore.

2. Système selon la revendication 1, dans lequel la pluralité de décalages Doppler ont une pluralité de directions, et dans lequel la détermination de la caractéristique de la zone d'ablation comporte l'identification d'une ou plusieurs régions du tissu qui correspondent respectivement à un ou plusieurs décalages Doppler de la pluralité de décalages Doppler.

3. Système selon la revendication 1, dans lequel les instructions, lorsqu'elles sont exécutées par le processeur, amènent en outre le dispositif informatique à :
déterminer une pluralité de caractéristiques de la pluralité de décalages Doppler,
la pluralité de caractéristiques comportant l'une quelconque ou une combinaison d'une quantité de décalage Doppler, d'une vitesse de changement de la quantité de décalage Doppler, d'une direction de décalage Doppler, d'une vitesse de changement de la direction de décalage Doppler, ou d'un emplacement dans le tissu sur la base duquel le décalage Doppler est détecté ; et à
déterminer si chacune de la pluralité de caractéristiques dépasse un ou plusieurs seuils, la détermination de la caractéristique de la zone d'ablation étant en outre sur la base d'un résultat de la détermination de si chacune de la pluralité de caractéristiques dépasse le ou les seuils.

4. Système selon la revendication 3, dans lequel la détermination de la caractéristique de la zone d'ablation comporte la définition d'une zone d'ablation,
les parties du tissu qui correspondent à la pluralité de décalages Doppler ayant des caractéristiques respectives qui dépassent le ou les seuils étant comprises dans la zone d'ablation, et
les parties du tissu qui correspondent à la pluralité de décalages Doppler ayant des caractéristiques respectives qui ne dépassent pas le ou les seuils étant exclues de la zone d'ablation,
la caractéristique de la zone d'ablation comportant une combinaison de la forme, de la taille et de l'emplacement de la zone d'ablation.

5. Système selon la revendication 1 ; dans lequel les instructions, lorsqu'elles sont exécutées par le processeur, amènent en outre le dispositif informatique à :
déterminer une caractéristique de la zone d'ablation prédite, si l'ablation continue comme prévu, sur la base de l'un quelconque ou d'une combinaison de la pluralité de décalages Doppler, de la caractéristique de la zone d'ablation déterminée ; d'un réglage du dispositif d'ablation, d'une durée écoulée pendant laquelle l'énergie micro-onde est distribuée au tissu, d'une durée attendue pendant laquelle l'énergie micro-onde sera distribuée au tissu, d'un emplacement dans le tissu auquel l'énergie micro-onde est distribuée, ou d'une ou plusieurs caractéristiques d'un signal par lequel l'énergie micro-onde est distribuée au tissu ; et à
amener le dispositif d'affichage à afficher, au moyen de l'interface utilisateur graphique, une représentation de la caractéristique de la zone d'ablation prédite.

6. Système selon la revendication 5, dans lequel la ou les caractéristiques du signal par lesquelles l'énergie micro-onde est distribuée comportent l'une quelconque ou une combinaison d'une quantité de puissance du signal, d'une amplitude du signal ; d'une fréquence du signal ; d'une fréquence de pulsation du signal, ou d'une largeur de pulsation du signal.

7. Système selon la revendication 1, dans lequel les instructions, lorsqu'elles sont exécutées par le processeur ; amènent en outre le dispositif informatique à :
déterminer une période de temps estimée jusqu'à l'achèvement d'une procédure d'ablation, sur la base de l'un quelconque ou d'une combinaison de la pluralité de décalages Doppler, de la caractéristique de la zone d'ablation déterminée, d'une durée écoulée pendant laquelle l'énergie micro-onde est distribuée au tissu, d'un emplacement dans le tissu auquel l'énergie micro-onde est distribuée ou d'une ou plusieurs caractéristiques d'un signal par lequel l'énergie micro-onde est distribuée ; et à
amener le dispositif d'affichage à afficher, par l'intermédiaire de l'interface utilisateur graphique, un indicateur de la période de temps estimée jusqu'à l'achèvement de la procédure d'ablation.

8. Système selon la revendication 1, dans lequel les instructions, lorsqu'elles sont exécutées par le processeur, amènent en outre le dispositif informatique à :
déterminer si une procédure d'ablation est achevée sur la base de l'un quelconque ou d'une combinaison de la pluralité de décalages Doppler, de la caractéristique de la zone d'ablation déterminée, d'une durée écoulée pendant laquelle l'énergie micro-onde est distribuée au tissu, ou d'un emplacement dans le tissu auquel l'énergie micro-onde est distribuée, ou d'une ou plusieurs caractéristiques d'un signal par lequel l'énergie micro-onde est distribuée ; et à amener le dispositif d'affichage à afficher, par l'intermédiaire de l'interface utilisateur graphique, un indicateur indiquant si la procédure d'ablation est achevée.

9. Système selon la revendication 8, dans lequel les instructions, lorsqu'elles sont exécutées par le processeur, amènent en outre le dispositif informatique à :
désactiver automatiquement la distribution de l'énergie micro-onde au tissu en réponse à une détermination que la procédure d'ablation est achevée.

10. Système selon la revendication 1, dans lequel les instructions, lorsqu'elles sont exécutées par le processeur, amènent en outre le dispositif informatique à :
déterminer un pourcentage d'achèvement d'une procédure d'ablation sur la base de l'un quelconque ou d'une combinaison de la pluralité de décalages Doppler, de la caractéristique de la zone d'ablation déterminée, d'une durée écoulée pendant laquelle l'énergie micro-onde est distribuée au tissu, d'un emplacement dans le tissu auquel l'énergie micro-onde est distribuée, ou d'une ou plusieurs caractéristiques d'un signal par lequel l'énergie micro-onde est distribuée ; et à
amener le dispositif d'affichage à afficher, par l'intermédiaire de l'interface utilisateur graphique, un indicateur du pourcentage d'achèvement de la procédure d'ablation.

11. Système selon la revendication 1, dans lequel l'énergie micro-onde amène le tissu à vibrer, et dans lequel les instructions, lorsqu'elles sont exécutées par le processeur, amènent le dispositif informatique à traiter les signaux de retour ultrasonores reçus pour identifier une région où le tissu vibre et amenant ainsi un nuage moucheté rouge et bleu détectable dans les signaux de retour d'ultrasons comme un décalage Doppler, le nuage moucheté rouge et bleu étant indicatif de la taille et de la forme de la zone d'ablation.
